# FASCICULE DE BREVET EUROPEEN

(11) **EP 4 616 892 B1**
(45) Date de publication et mention de la délivrance du brevet: **05.08.2026**
(21) Numéro de dépôt: 25161192.7
(22) Date de dépôt: 03.03.2025
(51) Int. Cl.: A61M 16/00, A61M 16/20

(54) **VENTILATEUR MÉDICAL AFFICHANT LA PRESSION D'OUVERTURE DES VOIES AÉRIENNES DU PATIENT**
MEDIZINISCHES BEATMUNGSGERÄT MIT ANZEIGE DES ÖFFNUNGSDRUCKS DER ATEMWEGE EINES PATIENTEN
MEDICAL VENTILATOR DISPLAYING OPENING PRESSURE OF PATIENT AIRWAY

(30) Priorité: 15.03.2024 FR 2402621
(43) Date de publication de la demande: 17.09.2025
(73) Titulaire: Air Liquide Medical Systems, 92182 Antony Cedex (FR)
(72) Inventeur: LESIMPLE, Arnaud, 92182 Antony Cedex (FR); POLARD, Laura, 92182 Antony Cedex (FR); LEFRANC, Mathilde, 92182 Antony Cedex (FR); RICHARD, Jean-Christophe, 92182 Antony Cedex (FR); DE BEAUFORT, Eloise, 92182 Antony Cedex (FR)
(74) Mandataire: Air Liquide

(56) Documents cités:
- WO-A1-2020/080986
- FR-A1- 2 887 777
- FR-A3- 3 137 296
- US-A- 5 738 090
- US-A1- 2011 023 881
- US-A1- 2013 255 682
- US-A1- 2015 100 917
- US-A1- 2019 275 276
- CHEN LU ET AL: "Potential for Lung Recruitment Estimated by the Recruitment-to-Inflation Ratio in Acute Respiratory Distress Syndrome. A Clinical Trial", AMERICAN THORACIC SOCIETY 2020 INTERNATIONAL CONFERENCE; MAY 15-20, 2020, vol. 201, no. 2, 15 January 2020 (2020-01-15), pages 178 - 187, XP093066439, ISSN: 1073-449X, DOI: 10.1164/rccm.201902-0334OC

## Description

L'invention concerne un ventilateur médical, c'est-à-dire un appareil d'assistance respiratoire apte à fourniture un gaz respiratoire, tel de l'air, de l'oxygène ou un mélange air/oxygène, permettant de déterminer et afficher la pression d'ouverture des voies aériennes du patient ou pression d'AOP (pour *Airways Opening Pressure* en anglais) d'un patient recevant le gaz respiratoire.

Le syndrome de détresse respiratoire aiguë (SDRA) est une cause fréquente de mortalité chez l'être humain. Sa prise en charge requiert l'application d'une ventilation dite « protectrice » chez les patients, laquelle est généralement mise en œuvre par un appareil d'assistance respiratoire, aussi appelé ventilateur médical, avec personnalisation des paramètres ventilatoires pour chaque patient, typiquement le débit, la pression et/ou la fréquence des cycles respiratoires.

Un phénomène dit de fermeture des voies aériennes a été signalé chez 23 à 52% des patients atteints du syndrome de détresse respiratoire aiguë (SDRA).

Dès lors, une détection de la fermeture des voies aériennes et une mesure de la pression d'ouverture des voies aériennes sont essentielles pour adapter la ventilation à chaque patient.

En effet, le réglage d'une pression expiratoire positive (PEP) inférieure à la pression d'ouverture des voies aériennes peut altérer la mesure de la mécanique respiratoire du patient.

En outre, des ouvertures et fermetures cycliques des petites voies respiratoires peuvent également se produire et favoriser des lésions pulmonaires induites par le ventilateur.

Il existe donc un risque pour les patients lorsque les paramètres de ventilation sont mal choisis ou de manière non-adéquate.

D'autres ventilateurs médicaux sont décrits par WO2020/080986, US5738090, FR3137296, US2011/023881, US2019/275276 et US2015/100917.

Un problème est dès lors de pouvoir déterminer une éventuelle fermeture des voies respiratoires chez les patients, notamment chez les patients SDRA, et de mesurer la pression d'ouverture des voies aériennes (AOP) lorsque cette fermeture des voies aériennes est présente afin d'éviter une mauvaise interprétation des courbes et des calculs de mécaniques respiratoires et par ailleurs d'opérer une personnalisation de la ventilation basée sur l'analyse du phénomène de fermeture des voies aériennes, de manière à éviter ou minimiser les risques pour les patients.

Une solution de l'invention concerne un ventilateur médical, i.e. un appareil d'assistance respiratoire/ventilatoire, permettant de fournir un gaz respiratoire à un patient comprenant :
- des moyens de fourniture de gaz pour fournir un gaz respiratoire,
- un circuit patient alimenté en gaz respiratoire par les moyens de fourniture de gaz,
- des moyens de mesure de pression configurés pour déterminer la pression du gaz dans le circuit patient,
- des moyens de détermination de débit configurés pour déterminer le débit de gaz respiratoire dans le circuit patient,
- des moyens de traitement de données comprenant au moins un (micro)processeur pour traiter les mesures de pression et/ou de débit opérées par les moyens de mesure de pression et les moyens de détermination de débit,
- des moyens d'affichage commandés par les moyens de traitement de données, et
- des moyens de mémorisation pour mémoriser une courbe pression/volume de compliance du circuit (P-Vcircuit_patient) représentant la compliance du circuit respiratoire.

De plus, le ventilateur médical comprend en outre des moyens de sélection, actionnables par l'utilisateur pour débuter une détermination de la pression d'ouverture des voies aériennes (AOP), et les moyens de traitement de données sont configurés pour opérer une détermination de la pression d'AOP en réponse à un actionnement par l'utilisateur des moyens de sélection :
a) en pilotant les moyens de fourniture de gaz pour fournir le gaz respiratoire à une fréquence respiratoire dite « basse » comprise entre 2 et 10 cycles ventilatoires par minute, et à un débit d'insufflation dit « lent » compris entre 2 et 10 L/min jusqu'à obtenir un volume inspiratoire compris entre 200 et 1500 mL ou une pression maximale (Pmax) comprise entre 20 et 60 cmH₂O,
b) en commandant un affichage sur les moyens d'affichage de la courbe pression/volume de compliance du circuit (P-Vcircuit_patient) mémorisée et d'une courbe pression/volume correspondant à l'insufflation à débit « lent » (P-Vdébit_lent), la courbe pression/volume d'insufflation à débit « lent » (P-Vcircuit_patient) mémorisée étant affichée de manière superposée à la courbe pression/volume de compliance du circuit (P-Vcircuit_patient),
c) en recherchant et localisant sur la courbe pression/volume d'insufflation à débit « lent » (P-Vdébit_lent), un point d'inflexion correspondant à un changement de pente de ladite courbe pression/volume d'insufflation à débit « lent » (P-Vdébit_lent) avec séparation de la courbe pression/volume d'insufflation à débit « lent » (P-Vdébit_lent) de la courbe pression/volume de compliance du circuit (P-Vcircuit_patient), et
d) en déterminant la valeur de pression au point d'inflexion localisé sur la courbe à débit « lent » (P-Vdébit_lent).

De plus, les moyens de traitement des données sont en outre configurés pour commander un affichage sur les moyens d'affichage, de la valeur de la pression d'AOP, i.e. la pression d'ouverture des voies aériennes du patient, correspondant à la valeur de pression ayant été déterminée au point d'inflexion présent de la courbe à débit « lent » (P-Vdébit_lent).

Dans le cadre de l'invention, afin de simplifier la compréhension, on utilise les abréviations et/ou simplifications suivantes :
- « pression d'AOP » pour désigner la pression d'ouverture des voies aériennes du patient ;
- « courbe de compliance » pour désigner la courbe pression/volume de compliance du circuit (P-Vcircuit_patient) ;
- « courbe à débit lent » pour désigner la courbe pression/volume à débit « lent » (P-Vdébit_lent).
- « %vol. » » signifie pourcentage en volume.
- « O₂ » désigne l'oxygène.
- les termes « moyens de» sont considérés comme totalement équivalents et substituables par les termes « dispositifs de », par exemple les termes « moyens de traitement d'informations » peuvent être remplacés par « dispositif de traitement d'informations », les termes « moyens d'affichage » peuvent être remplacés par « dispositif d'affichage », les « moyens de mémorisation» peuvent être remplacés par « dispositif de mémorisation» ...

Selon le mode de réalisation considéré, le ventilateur médical de l'invention peut comprendre l'une ou plusieurs des caractéristiques suivantes :
- le circuit patient comprend une branche inspiratoire et une branche expiratoire.
- les moyens de fourniture de gaz fournissent le gaz respiratoire au débit d'insufflation pendant une durée suffisante pour obtenir le volume inspiratoire compris entre 200 et 1500 mL désiré ou la pression maximale (Pmax) comprise entre 20 et 60 cmH₂O souhaitée.
- ladite durée suffisante découle, i.e. dépend ou est fonction, du réglage du débit et du volume inspiratoire désiré.
- ladite durée suffisante est préférentiellement inférieure à 60 secondes, par exemple comprise typiquement entre 1 et 45 secondes.
- la branche inspiratoire et la branche expiratoire sont raccordées fluidiquement à une pièce de liaison, typiquement une pièce en Y.
- le circuit patient est raccordée fluidiquement à une interface respiratoire, tel un masque respiratoire, une sonde d'intubation endotrachéale ou autre.
- la branche inspiratoire fournit le gaz respiratoire à l'interface respiratoire.
- le gaz respiratoire contient de l'oxygène (O₂) en une proportion d'au moins 20%vol., de préférence au moins 21%vol. environ.
- le gaz respiratoire est de l'air, de l'oxygène ou un mélange air/oxygène.
- la branche inspiratoire est reliée fluidiquement aux moyens de fourniture de gaz, en particulier à la sortie de fourniture de gaz d'une turbine motorisée.
- la branche expiratoire récupère le gaz expiré par le patient dans l'interface respiratoire, c'est-à-dire le gaz contenant du CO₂.
- les moyens de fourniture de gaz respiratoire comprennent une source de gaz respiratoire, telle une turbine motorisée.
- alternativement, les moyens de fourniture de gaz respiratoire comprennent des moyens de raccordement fluidique à une source de gaz respiratoire, telle une prise de gaz murale.
- les moyens de traitement de données comprenant au moins un (micro)processeur pour déterminer un ou plusieurs volumes gazeux entrant ou sortant du ventilateur à partir du traitement opéré sur les mesures de débit opérées par les moyens de détermination de débit.
- les moyens de mesure de pression comprennent un capteur de pression.
- les moyens de détermination de débit comprennent un capteur de débit.
- les moyens de mesure de pression sont agencés dans la branche inspiratoire du circuit patient.
- les moyens de mesure de pression sont agencés à l'intérieur du ventilateur, i.e. dans sa carcasse.
- les moyens d'affichage sont configurés pour opérer des affichages, notamment une ou des courbes, valeurs numériques, textes, messages, fenêtres, icônes.... ou toute autre information.
- les moyens de traitement des données sont en outre configurés pour commander un affichage sur les moyens d'affichage, d'une information relative à l'existence d'une fermeture des voies aériennes du patient, i.e. en particulier lorsqu'un point d'inflexion est détecté et une AOP est déterminée, par exemple un message sous forme de texte indiquant la présence/existence ou non d'une fermeture des voies aériennes, comme par exemple "présence d'une fermeture des voies aériennes" ou "fermeture des voies aériennes détectée" ou, à l'inverse, "absence de fermeture des voies aériennes" ou "voies aériennes ouvertes", ou tout autre message équivalent.
- les moyens de traitement des données sont en outre configurés pour commander un affichage sur les moyens d'affichage d'un message sous forme de texte indiquant la valeur de pression « x » correspondant à la AOP déterminée, c'est-à-dire en cas de présence de fermeture des voies aériennes, par exemple " AOP = x cmH₂O", avec x typiquement compris entre 1 et 30 cmH₂O.
- des moyens de traitement de données comprenant au moins un (micro)processeur mettant en œuvre un ou des algorithmes.
- les moyens de traitement de données comprennent un microcontrôleur.
- ledit au moins un (micro)processeur est agencé sur une carte électronique.
- les moyens de traitement de données sont configurés pour déterminer le volume inspiré et le volume expiré
- les moyens d'affichage comprennent un écran tactile, c'est-à-dire à dalle tactile.
- les moyens de sélection comprennent une touche virtuelle affichée sur les moyens d'affichage, en particulier sur l'écran tactile, c'est-à-dire une touche tactile, i.e. à actionnement par appui ou pression digitale.
- l'écran tactile est à affichage en couleurs.
- la fréquence respiratoire « basse » est comprise entre 3 et 7 cycles ventilatoires par minute, par exemple de l'ordre de 5 cycles ventilatoires par minute.
- le débit d'insufflation « lent » est compris entre 3 et 7 L/min, par exemple de l'ordre de 5 L/min.
- les moyens de traitement de données sont configurés pour commander un affichage de la valeur de la pression d'AOP sous forme d'une valeur positive de pression, de préférence exprimée en cmH₂O ou en une autre unité.
- la courbe à débit lent est obtenue à partir des valeurs de pressions et débit traitées par le (les) microprocesseur.
- la courbe à débit « lent » est une courbe affichée et mise à jour en temps réel.
- la compliance (e.g. la courbe de compliance mémorisée) est obtenue avant début de traitement du patient.
- la compliance du circuit patient est mémorisée avant le début de traitement du patient lors des tests permettant de vérifier le bon fonctionnement de la machine ou "auto tests". Cette compliance du circuit est ensuite utilisée pour déterminer et afficher la courbe de compliance (i.e. courbe P-Vcircuit_patient) superposée à la courbe à débit lent (i.e. courbe P-Vdébit_lent).
- les moyens de traitement des données sont en outre configurés pour déterminer une pression de PEP à partir de la pression d'AOP ayant été déterminée.
- les moyens de traitement des données sont en outre configurés pour recommander (i.e. déterminer) une pression de PEP supérieure à la pression d'AOP ayant été déterminée.
- les moyens de traitement des données sont en outre configurés pour commander un affichage sur les moyens d'affichage de la pression de PEP recommandée.
- la valeur de pression d'AOP est comprise entre 1 et 30 cmH₂O.
- les moyens de fourniture de gaz comprennent une turbine motorisée pilotée par les moyens de traitement des données.
- les moyens de mesure de pression comprennent un capteur de pression et/ou les moyens de détermination de débit comprennent un capteur de débit.
- il comprend une valve de PEP agencée sur le circuit patient, en particulier sur la branche expiratoire.
- les moyens de traitement des données sont configurés pour régler la pression de PEP de ladite valve de PEP à la pression de PEP recommandée.
- les moyens de traitement des données sont configurés pour régler la pression de PEP de ladite valve de PEP en réponse à l'activation par l'utilisateur d'un moyen de confirmation de réglage, de préférence après appui digital de l'utilisateur sur une touche de confirmation virtuelle affichée sur l'écran digital.
- il comprend des moyens d'alimentation en courant électrique alimentant le ventilateur, tel un raccordement au secteur (110/220V) et/ou une batterie rechargeable.

Selon un autre aspect (non revendiqué), la présente divulgation concerne aussi un procédé de pilotage d'un ventilateur médical selon l'invention pour déterminer et afficher une valeur de pression d'AOP, en particulier un ventilateur médical tel que décrit ci-avant, dans lequel :
A. on opère une détermination de la pression d'ouverture des voies aériennes, appelée « pression d'AOP », en :
   I. fournissant le gaz respiratoire à une fréquence respiratoire dite « basse » comprise entre 2 et 10 cycles ventilatoires par minute, et à un débit d'insufflation dit « lent » compris entre 2 et 10 L/min, jusqu'à obtenir un volume inspiratoire compris entre 200 et 1500 mL ou une pression maximale (Pmax) comprise entre 20 et 60 cmH₂O,
   II. affichant de la courbe pression/volume de compliance du circuit mémorisée (C1) et d'une courbe pression/volume correspondant à l'insufflation à débit lent (C2), la courbe pression/volume (C2) d'insufflation à débit lent étant affichée de manière superposée à la courbe pression/volume de compliance (C1) du circuit mémorisée,
   III. recherchant et localisant sur la courbe pression/volume (C2) d'insufflation à débit lent, un point d'inflexion (PI) correspondant à un changement de pente de ladite courbe pression/volume (C2) d'insufflation à débit lent avec séparation de la courbe pression/volume d'insufflation à débit lent (C2) de la courbe pression/volume de compliance du circuit (C1), et
   IV. déterminant la valeur de pression au point d'inflexion (PI) localisé sur la courbe à débit lent (C1), et
B. on affiche la valeur de pression d'AOP correspondant à la valeur de pression au point d'inflexion (PI) ayant été déterminée sur la courbe à débit lent (C2).

Selon le mode de réalisation considéré, le procédé peut comprendre l'une ou plusieurs des caractéristiques suivantes :
- on débute la détermination de la pression d'AOP en réponse à un actionnement par l'utilisateur de moyens de sélection.
- on fournit le gaz respiratoire à la fréquence respiratoire dite « basse » et au débit d'insufflation dit « lent » en pilotant les moyens de fourniture de gaz.
- l'affichage de la courbe pression/volume de compliance du circuit mémorisée (C1) et de la courbe pression/volume correspondant à l'insufflation à débit lent (C2) est réalisé sur les moyens d'affichage.
- on affiche la valeur de pression d'AOP sur les moyens d'affichage.
- le ventilateur médical comprend des moyens de fourniture de gaz alimentant un circuit patient en gaz respiratoire, comprenant des moyens de mesure de pression et des moyens de détermination de débit pour déterminer la pression et le débit du gaz dans le circuit patient ; des moyens de traitement de données comprenant au moins un processeur pour traiter lesdites mesures de pression et/ou de débit ; des moyens d'affichage ; et des moyens de mémorisation mémorisant une courbe pression/volume de compliance du circuit (C1) représentant la compliance du circuit respiratoire.
- le gaz respiratoire est de l'air, de l'oxygène ou un mélange air/oxygène.
- on fournit le gaz respiratoire via des moyens de fourniture de gaz pilotés par les moyens de traitement des données.
- les moyens de fourniture de gaz comprennent une turbine motorisée.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un ventilateur médical selon l'invention.
Fig. 2 schématise un mode de réalisation de l'affichage des courbes pression/volume superposées montrant le point d'inflexion apparaissant en cas de fermeture des voies aériennes du patient.

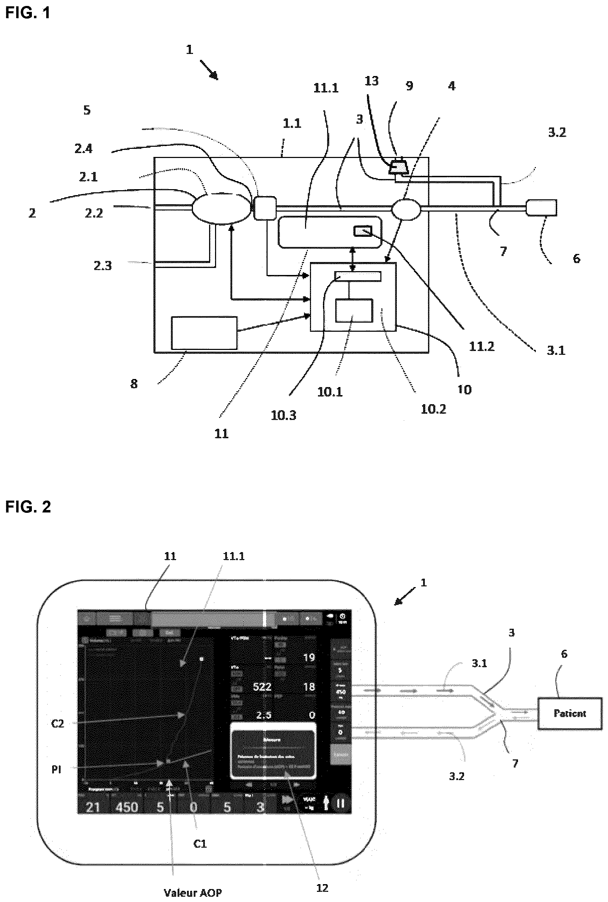

Fig. 1 schématise un mode de réalisation d'un ventilateur médical 1 selon l'invention, lequel comprend des moyens de fourniture de gaz 2 pour fournir un gaz respiratoire à un patient en ayant besoin, typiquement une personne souffrant de SDRA.

Ici, les moyens de fourniture de gaz 2 comprennent une turbine motorisée 2.1 alimentée en air par un conduit d'amenée d'air 2.2 relié à l'atmosphère ambiante et en oxygène par un conduit d'amenée d'oxygène 2.3 pouvant être raccordé à une source d'oxygène (non montrée), telle une bouteille d'oxygène sous pression ou une prise murale hospitalière alimentée par une conduite d'amenée d'oxygène.

Toutefois, selon un autre mode de réalisation (non montré), les moyens de fourniture de gaz 2 comprennent des moyens de raccordement fluidique à une source de gaz respiratoire, telle une prise de gaz murale, et une ou des vannes pilotées, telles des électrovannes pilotées par les moyens de traitement de données 10.

Typiquement, le gaz respiratoire fourni par les moyens de fourniture de gaz 2 est de l'air, de l'oxygène ou un mélange gazeux air/oxygène (>20%vol. O₂, de préférence > 21% vol. O₂ environ).

Les moyens de fourniture de gaz 2 alimentent un circuit patient 3 comprenant une branche inspiratoire 3.1 et une branche expiratoire 3.2, tels des passages ou des conduits de gaz, notamment des tuyaux flexibles, raccordées fluidiquement à une interface respiratoire 6, tel un masque respiratoire ou une sonde endotrachéale, via une pièce de liaison 6, à savoir une pièce en Y ou analogue.

La branche inspiratoire 3.1 amène le gaz respiratoire à l'interface respiratoire 6, lequel gaz est inspiré par le patient, lors de ses phases inspiratoires, alors que la branche expiratoire 3.2 convoie les gaz chargés de CO₂ expirés par le patient dans l'interface respiratoire 6, lors des phases expiratoires. La branche expiratoire 3.2 est reliée à une sortie d'évacuation 9 des gaz expirés du ventilateur 1 reliée fluidiquement à l'atmosphère ambiante.

Une valve de PEP 13 est agencée sur le circuit patient 3, en particulier sur la branche expiratoire 3.2. Les moyens de traitement de données sont configurés pour régler la pression de PEP de ladite valve de PEP 13 à la pression de PEP proposée, comme expliqué ci-après, en particulier en réponse à l'activation par l'utilisateur d'un moyen de confirmation de réglage, de préférence après appui digital de l'utilisateur sur une touche de confirmation virtuelle affichée sur l'écran digital 11.1.

Par ailleurs, le circuit patient 3, en particulier la branche inspiratoire 3.1, comprend des moyens de mesure de pression 4, tel un capteur de pression, permettant de mesurer, i.e. déterminer, la pression du gaz dans la branche inspiratoire 3.1, et des moyens de détermination de débit 5, tel un capteur de débit, servant à déterminer le débit de gaz respiratoire dans le circuit patient 3.

De préférence, les moyens de détermination de débit 5 sont agencés à proximité de la sortie 2.4 des moyens de fourniture de gaz 2, c'est-à-dire immédiatement en aval de la turbine 2.1.

Par ailleurs, les moyens de mesure de pression 4 sont situés en amont de la pièce de liaison 6, i.e. la pièce en Y.

Le ventilateur médical 1 comprend des moyens de traitement de données 10, aussi appelés moyens de pilotage, comprenant un (ou des) microprocesseur 10.1 agencé sur une carte électronique 10.2, lesquels permettent de traiter des données et de piloter ou commander des éléments du ventilateur 1, notamment le fonctionnement de la turbine 2.1, les affichages sur l'écran d'affichage 11, de préférence un écran à dalle tactile 11.1, ou autres.

Les moyens de mesure de pression 4 et les moyens de détermination de débit 5, i.e. les capteurs de pression et de débit, sont reliés électriquement aux moyens de traitement de données 10, typiquement au (micro)processeur 10.1, afin de leur fournir des mesures de pression et de débit (i.e. valeurs ou signaux), et les moyens de traitement de données 10 sont configurés pour traiter les mesures de pression et de débit provenant des moyens de mesure de pression 4 et les moyens de détermination de débit 5, i.e. les capteurs de pression et de débit

Les moyens de traitement de données 10 peuvent par exemple en déduire un ou des volumes gazeux entrant ou sortant du ventilateur 1, typiquement du circuit patient 3, c'est-à-dire du gaz respiratoire fourni au patient ou des gaz riches en CO₂ expirés par le patient.

Le ventilateur 1 comprend par ailleurs des moyens de mémorisation 10.3, typiquement une mémoire informatique, telle une mémoire flash, RAM ou analogue, servant à mémoriser une courbe (i.e. on mémorise une courbe ou des valeurs de compliance permettant d'établir une telle courbe) pression/volume de compliance du circuit (P-Vcircuit_patient) représentant la compliance du circuit respiratoire (i.e. courbe C1 sur Fig. 1) ou des valeurs telle que la valeur d'AOP. Les moyens de mémorisation 10.3 sont préférentiellement agencés sur la carte électronique 10.2 et reliés électriquement au (micro)processeur 10.1

Des moyens d'alimentation en courant électrique, tel un raccordement au secteur (110/220V), par exemple des câbles électriques, et/ou une batterie rechargeable, alimentent le ventilateur 1, en particulier la turbine 2.1, les capteurs 4, 5, les moyens de traitement de données 10, l'écran tactile 11.... et les autres composants nécessitant du courant électrique pour fonctionner.

Tout ou partie des différents composants sont agencés dans une carcasse ou coque externe 1.1 du ventilateur 1.

Selon l'invention, on souhaite pouvoir déterminer une éventuelle fermeture des voies respiratoires chez les patients, tels des patients SDRA, lesquels sont ventilés avec un ventilateur médical 1, tel celui décrit ci-avant, et de mesurer la pression d'ouverture de leurs voies aériennes (i.e. la pression d'AOP) lorsqu'elle est présente, afin d'éviter une mauvaise interprétation des courbes et des calculs de mécaniques respiratoires et par ailleurs d'opérer une personnalisation de la ventilation basée sur l'analyse du phénomène de fermeture des voies aériennes, et de manière à éviter ou minimiser les risques pour les patients.

Pour ce faire, le ventilateur 1 est doté de moyens de sélection 11.2, actionnables par l'utilisateur pour débuter une détermination de la pression d'ouverture des voies aériennes ou pression d'AOP, c'est-à-dire pour lancer la procédure de détermination d'une éventuelle fermeture des voies aériennes du patient de la pression d'AOP correspondante, comme décrit ci-après.

De préférence, les moyens de sélection 11.2 comprennent une touche de sélection, en particulier une touche virtuelle, i.e. une touche tactile, affichée sur l'écran tactile 11.1 sur laquelle l'utilisateur vient appuyer avec un doigt, tel son index, pour lancer la procédure de détermination d'une pression d'AOP.

La touche de sélection peut être affichée en permanence sur l'écran 11.1 ou s'afficher uniquement après que l'utilisateur, i.e. un personnel soignant, ait effectué un choix de procédure de détermination de pression d'AOP au sein d'un menu dédié affiché sur l'écran 11.1.

Selon un autre mode de réalisation, le ventilateur 1 peut aussi comporter un bouton spécifique, par exemple agencé sur sa carcasse 1.1, dont l'actionnement engendre un démarrage de la procédure de détermination de la pression d'ouverture des voies respiratoires, i.e. de la pression d'AOP.

Dans tous les cas, en réponse à un tel actionnement par l'utilisateur des moyens de sélection 11.2, les moyens de traitement de données 10, typiquement le processeur 10.1, sont configurés pour opérer une détermination d'une éventuelle fermeture des voies aériennes du patient et, lorsqu'elles sont fermées, une détermination de la pression d'AOP correspondante, c'est-à-dire lancer une procédure de détermination de pression d'AOP.

Pour ce faire, les moyens de traitement de données 10 pilotent d'abord les moyens de fourniture de gaz respiratoire 2, telle la turbine motorisée 2.1, pour fournir le gaz respiratoire (i.e. teneur en O₂ > 20%vol.), par exemple de l'air, de l'O₂ ou un mélange air/O₂, à une fréquence respiratoire dite « basse » comprise entre 2 et 10 cycles ventilatoires par minute, par exemple de l'ordre de 5 cycles ventilatoires par minute, et à un débit d'insufflation dit « lent » compris entre 2 et 10 L/min, par exemple de l'ordre de 5 L/min, et ce, pendant une durée suffisante pour obtenir un volume inspiratoire compris entre 200 et 1500 mL ou une pression maximale (Pmax) comprise entre 20 et 60 cmH₂O. Les réglages de débit et de volume inspiratoire déterminent la durée. En général, cette durée est inférieure à 60 secondes, par exemple de l'ordre de 1 sec à 45 sec.

Par ailleurs, les moyens de traitement de données 10 commandent un affichage sur les moyens d'affichage 11 de la courbe pression/volume de compliance (courbe C1 sur Fig. 2) du circuit (P-Vcircuit_patient) ayant été préalablement mémorisée par les moyens de mémorisation 11.3 et d'une courbe pression/volume (courbe C2 sur Fig. 2) correspondant à l'insufflation à débit « lent » (P-Vdébit_lent) déterminée pendant la durée donnée.

La courbe pression/volume d'insufflation à débit « lent » (P-Vcircuit_patient) (i.e. courbe C2), plus simplement appelée « courbe à débit lent », est affichée de manière superposée à la courbe pression/volume de compliance (P-Vcircuit_patient) mémorisée (i.e. courbe C1), plus simplement appelée « courbe de compliance », comme illustré en Fig. 2.

Concernant la courbe de compliance (P-Vcircuit_patient), elle est préalablement déterminée, par exemple, lors d'une procédure d'autotests ou analogue du ventilateur 1. La compliance du circuit patient est mémorisée avant le début de traitement du patient lors des tests permettant de vérifier le bon fonctionnement de la machine ou "auto tests". Cette compliance du circuit est ensuite utilisée pour déterminer et afficher la courbe de compliance, i.e. courbe P-Vcircuit_patient, de manière superposée à la courbe à débit lent, i.e. courbe P-Vdébit_lent.

En effet, une procédure d'autotests d'un ventilateur 1 est généralement effectuée avant de ventiler, i.e. traiter, un patient afin de vérifier le bon fonctionnement des capteurs ou autres composants, et de prendre en compte les caractéristiques du circuit patient 3 du ventilateur 1.

Pour ce faire, un bouchon d'obturation peut être inséré dans le circuit patient 3, typiquement au niveau de la pièce en Y 7 du circuit patient 3 de manière à empêcher le flux de gaz respiratoire, tel de l'air, délivré par les moyens de fourniture de gaz 2 de sortir du circuit 3 par l'interface respiratoire 6, e.g. masque 6 ou analogue.

Ensuite, on lance la procédure d'autotests proprement dite, par exemple via un menu de sélection spécifique s'affichant sur l'écran 11 du ventilateur 1.

Celle-ci comprend une insufflation d'un débit donné de gaz respiratoire dans la branche inspiratoire 3.1 du circuit patient 3 avec mesure de la pression correspondante par le capteur de pression 4, jusqu'à l'atteinte d'une pression maximale réglée (Pmax), par exemple de l'ordre de 60 cmH2O.

En procédant à une intégration du débit gazeux, on peut alors calculer le volume de gaz (Vcircuit_patient) délivré dans le circuit patient 3.

Les moyens de traitement de données 10, typiquement le processeur 10.1, peuvent dès lors déterminer la compliance du circuit (Ccircuit_patient) en utilisant les valeurs de pression maximale réglée (Pmax) et de volume de gaz délivré ainsi déterminé (Vcircuit_patient), en opérant le calcul suivant : Ccircuit_patient = Vcircuit_patient / Pmax.

En fait, on considère que la compliance est linéaire, donc on ne prend que deux points de mesure de débit et de pression (dont le point à débit et pression nulle). Le débit insufflé est constant pour obtenir le volume Vcircuit_patient.

Une fois la compliance du circuit déterminée ainsi que d'autres procédures de vérifications internes, la procédure d'autotests est stoppée et le bouchon d'obturation peut être retiré afin de pouvoir utiliser le ventilateur 1 pour traiter un patient.

Les moyens de traitement de données 10, typiquement le processeur 10.1, sont par ailleurs configurés pour rechercher et localiser sur la courbe à débit lent, i.e. courbe C2 sur Fig. 2, tout point d'inflexion PI à partir duquel il se produit un changement de pente de la courbe à débit lent, (P-Vdébit_lent) accompagné d'une séparation de la courbe à débit lent (i.e. courbe C2) de la courbe de compliance du circuit (P-Vcircuit_patient) (i.e. courbe C1), c'est-à-dire un écartement brusque des deux courbes C1, C2 l'une de l'autre, comme illustré sur Fig. 2.

Si aucun point d'inflexion PI n'est trouvé, cela signifie qu'il n'y a pas de fermeture des voies aériennes du patient à ce niveau de PEP ou alors qu'elle n'est pas visible à cette pression. A l'inverse, si un point d'inflexion PI, i.e. point d'inflexion PI sur Fig. 2, est trouvé et localisé sur la courbe à débit lent C2, cela signifie que le patient considéré fait l'objet d'une fermeture de ses voies aériennes et la pression d'AOP correspond alors à la valeur de pression au point d'inflexion PI.

Les moyens de traitement de données 10, typiquement le processeur 10.1, sont dès lors configurés pour déterminer la valeur de pression au point d'inflexion PI ayant été localisé sur la courbe pression/volume d'insufflation à débit « lent » (P-Vdébit_lent), i.e. C2 sur Fig. 2.

Une fois déterminée, la valeur de pression au point d'inflexion PI ayant été déterminée est affichée sur l'écran d'affichage 11.1 des moyens d'affichage 11, étant donné que la pression d'AOP correspondant à la valeur de pression au point d'inflexion PI de la courbe à débit « lent » (P-Vdébit_lent) ), i.e. C2 sur Fig. 2.

Fig. 2 schématise un exemple de monitorage en temps réel des courbes pression/volume C1, C2 obtenues à partir des valeurs de pressions et débit traités par le microprocesseur 10.1, à savoir la courbe à débit lent (P-Vdébit_lent), désignée par « C2 », et de la courbe de compliance (P-Vcircuit_patient), désignée par « C1 », obtenue à partir des valeurs de pressions traitées par le microprocesseur 10.1 et de la compliance du circuit mesurée lors d'une procédure d'autotests, comme expliqué ci-avant.

En particulier, il est important que le gaz soit insufflé à débit lent lors de l'établissement de la courbe à débit lent car cela permet de supprimer la composante résistive de la pression des voies aériennes, rendant alors possible la détection d'une fermeture des voies aériennes du patient par le biais d'une localisation sur les courbes (C1, C2) de tout changement abrupt de pente, c'est-à-dire du point d'inflexion PI présent en cas de fermeture des voies aériennes mais absent lorsque les voies aériennes du patient ne sont pas fermées, lequel point d'inflexion PI permet de déterminer la pression d'AOP.

Sur le graphique de Fig. 2, l'axe des abscisses donnent des valeurs de pression, exprimées en cmH2O, et l'axe des ordonnées donnent des valeurs de volume, exprimées en mL. Bien entendu, d'autres unités/grandeurs pourraient être utilisées.

On voit qu'un point d'inflexion PI apparait là où les deux courbes C1, C2 s'écartent brusquement l'une de l'autre, ce qui indique une fermeture des voies aériennes du patient considéré et donc l'existence d'une pression d'AOP.

La valeur de pression d'AOP correspond alors à la valeur positive de pression au point d'inflexion PI, à savoir ici une pression de 22,9 cmH₂O, comme indiqué par l'axe des abscisses.

Ces informations, à savoir l'existence d'une fermeture des voies aériennes et la valeur de la pression d'AOP, sont alors affichées dans une fenêtre d'affichage 12 dédiée de l'écran d'affichage 11.1. L'affichage est commandé par les moyens de traitement de données 10, en particulier par le processeur 10.1.

L'affichage au sein de la fenêtre d'affichage 12 peut comprendre différentes informations utiles au personnel soignant, à savoir notamment :
- un message sous forme de texte indiquant la présence/existence ou non d'une fermeture des voies aériennes, comme par exemple "présence d'une fermeture des voies aériennes" ou "absence de fermeture des voies aériennes" ; et/ou
- un message sous forme de texte indiquant la valeur « x » de la pression d'AOP en cas de présence de fermeture des voies aériennes, par exemple "AOP = x cmH₂O", avec x compris entre 1 et 30 cmH₂O.

Préférentiellement, les moyens de traitement des données 10 peuvent aussi être configurés pour déterminer et proposer une stratégie ventilatoire, c'est-à-dire une action à mener par le personnel soignant, en fonction de la valeur de la pression d'AOP du patient ayant été déterminée.

Ainsi, si la PEP de la valve de PEP 13 est réglée à une valeur inférieure à la valeur de la pression d'AOP, les moyens de traitement des données 10 peuvent proposer une modification du réglage de la PEP à une valeur supérieure à celle de la valeur de la pression d'AOP (à considérer de préférence en association avec d'autres données cliniques disponibles) et commander un affichage de cette recommandation de réglage de la PEP sur les moyens d'affichage 11.

Par exemple, les moyens de traitement des données 10 peuvent commander un affichage d'un message (sous forme de texte) de recommandation de réglage de la PEP, comme par exemple "conservation de la PEP" ou "augmentation de la PEP", soit dans la fenêtre 12, soit ailleurs sur l'écran 11.1. La valeur de PEP recommandée est aussi avantageusement affichée.

Grâce à l'invention, en connaissant la valeur de pression d'AOP, le personnel soignant peut régler la pression expiratoire positive ou PEP de la valve de PEP 13 à une valeur au-dessus de la pression d'AOP afin de pouvoir maintenir les voies aériennes ouvertes et ce, afin de limiter les risques de lésions pulmonaires chez le patient.

D'une façon générale, selon l'invention, la détection de la fermeture des voies aériennes du patient et la détermination de la pression d'ouverture des voies aériennes (pression d'AOP) par analyse de la courbe de compliance et de la courbe à débit lent (C1, C2), comme expliqué ci-dessus, se font de manière automatique au sein du ventilateur médical 1.

Un ventilateur médical 1 selon l'invention est particulièrement bien adapté au traitement de patients, i.e. hommes, femmes ou enfants, par exemple de patients atteints du syndrome de détresse respiratoire aiguë (SDRA). En effet, il permet d'opérer un monitorage et une détection, en temps réel, du phénomène de fermeture des voies aériennes et une détermination de la pression d'ouverture des voies aériennes (AOP) des patients ventilés, tels les patients atteints de SDRA.

## Revendications

1. Ventilateur médical (1) comprenant :
- des moyens de fourniture de gaz (2) pour fournir un gaz respiratoire,
- un circuit patient (3) alimenté en gaz respiratoire par les moyens de fourniture de gaz (2),
- des moyens de mesure de pression (4) configurés pour déterminer la pression du gaz dans le circuit patient (3),
- des moyens de détermination de débit (5) configurés pour déterminer le débit de gaz respiratoire dans le circuit patient (3),
- des moyens de traitement de données (10) comprenant au moins un processeur (10.1) pour traiter les mesures de pression et/ou de débit opérées par les moyens de mesure de pression (4) et les moyens de détermination de débit (5),
- des moyens d'affichage (11) commandés par les moyens de traitement de données (10), et
- des moyens de mémorisation (10.3) pour mémoriser une courbe pression/volume de compliance du circuit (C1) représentant la compliance du circuit respiratoire,
**caractérisé en ce que** :
- il comprend en outre des moyens de sélection (11.2), actionnables par l'utilisateur pour débuter une détermination de la pression d'ouverture des voies aériennes (ou pression d'AOP),
- les moyens de traitement de données (10) sont configurés pour opérer une détermination de la pression d'AOP en réponse à un actionnement par l'utilisateur des moyens de sélection (11.2) :
a) en pilotant les moyens de fourniture de gaz (2) pour fournir le gaz respiratoire à une fréquence respiratoire dite « basse » comprise entre 2 et 10 cycles ventilatoires par minute, et à un débit d'insufflation dit « lent » compris entre 2 et 10 L/min, jusqu'à obtenir un volume inspiratoire compris entre 200 et 1500 mL ou une pression maximale (Pmax) comprise entre 20 et 60 cmH₂O,
b) en commandant un affichage sur les moyens d'affichage (11) de la courbe pression/volume de compliance du circuit mémorisée (C1) et d'une courbe pression/volume correspondant à l'insufflation à débit lent (C2), la courbe pression/volume (C2) d'insufflation à débit lent étant affichée de manière superposée à la courbe pression/volume de compliance (C1) du circuit mémorisée,
c) en recherchant et localisant sur la courbe pression/volume (C2) d'insufflation à débit lent, un point d'inflexion (PI) correspondant à un changement de pente de ladite courbe pression/volume (C2) d'insufflation à débit lent avec séparation de la courbe pression/volume d'insufflation à débit lent (C2) de la courbe pression/volume de compliance du circuit (C1), et
d) en déterminant la valeur de pression au point d'inflexion (PI) localisé sur la courbe à débit lent (C1),
- et les moyens de traitement des données (10) sont en outre configurés pour commander un affichage sur les moyens d'affichage (11), de la valeur de pression d'AOP (20) correspondant à la valeur de pression au point d'inflexion (PI) ayant été déterminée sur la courbe à débit lent (C2).

2. Ventilateur selon la revendication 1, **caractérisée en ce que** les moyens de traitement des données (10) sont en outre configurés pour commander un affichage sur les moyens d'affichage (11), d'une information relative à l'existence d'une fermeture des voies aériennes du patient.

3. Ventilateur selon la revendication 1, **caractérisée en ce que** les moyens d'affichage (11) comprennent un écran tactile (11.1).

4. Ventilateur selon la revendication 1, **caractérisée en ce que** les moyens de sélection (11.2) comprennent une touche virtuelle affichée sur les moyens d'affichage (11), en particulier sur l'écran tactile (11.1).

5. Ventilateur selon la revendication 1, **caractérisée en ce que** les moyens de traitement des données (10) sont en outre configuré pour commander un affichage sur les moyens d'affichage (11) d'une pression de PEP recommandée, ladite pression de PEP recommandée étant déterminée à partir de la pression d'AOP (20) ayant été déterminée.

6. Ventilateur selon la revendication 1, **caractérisée en ce que** la valeur de pression d'AOP (20) est comprise entre 1 et 30 cmH₂O.

7. Ventilateur selon la revendication 1, **caractérisée en ce que** les moyens de fourniture de gaz (2) comprennent une turbine motorisée (2.1) pilotée par les moyens de traitement des données (10).

8. Ventilateur selon la revendication 1, **caractérisée en ce que** les moyens de mesure de pression (4) comprennent un capteur de pression.

9. Ventilateur selon la revendication 1, **caractérisée en ce que** les moyens de détermination de débit (5) comprennent un capteur de débit.

10. Ventilateur selon les revendications 1 et 5, **caractérisée en ce qu'**il comprend une valve de PEP (13) agencée sur le circuit patient (3) et les moyens de traitement des données (10) sont configurés pour régler la pression de PEP de ladite valve de PEP (13) à la pression de PEP recommandée.

11. Ventilateur selon la revendication 1, **caractérisée en ce que** les moyens de traitement des données (10) sont configurés pour piloter les moyens de fourniture de gaz (2) pour fournir le gaz respiratoire à une fréquence respiratoire dite « basse » comprise entre 3 et 7 cycles ventilatoires par minute et/ou à un débit d'insufflation dit « lent » compris entre 3 et 7 L/min.

12. Ventilateur selon la revendication 5, **caractérisée en ce que** la pression de PEP recommandée est supérieure à la pression d'AOP (20) ayant été déterminée.

13. Ventilateur selon la revendication 1, **caractérisée en ce que** les moyens de fourniture de gaz (2) sont configurés pour fournir un gaz respiratoire choisi parmi l'air, l'oxygène et un mélange air/oxygène.

14. Ventilateur selon la revendication 1, **caractérisée en ce que** les moyens de fourniture de gaz fournissent le gaz respiratoire au débit d'insufflation pendant une durée suffisante pour obtenir le volume inspiratoire compris entre 200 et 1500 mL désiré ou la pression maximale (Pmax) comprise entre 20 et 60 cmH₂O souhaitée.

15. Ventilateur selon la revendication 14, **caractérisée en ce que** ladite durée suffisante est inférieure à 60 secondes.

## Patentansprüche

1. Medizinisches Beatmungsgerät (1) mit:
- einer Gasversorgungsvorrichtung (2) zur Bereitstellung eines Atemgases,
- einen Patientenkreislauf (3), der von den Gasversorgungsmitteln (2) mit Atemgas versorgt wird,
- Druckmessmittel (4), die so konfiguriert sind, dass sie den Gasdruck im Patientenkreislauf (3) bestimmen,
- Durchflussermittlungsmittel (5), die zur Ermittlung des Atemgasdurchflusses im Patientenkreislauf (3) ausgelegt sind,
- Datenverarbeitungsmittel (10), die mindestens einen Prozessor (10.1) umfassen, um die von den Druckmessmitteln (4) und den Durchflussbestimmungsmitteln (5) durchgeführten Druck- und/oder Durchflussmessungen zu verarbeiten,
- Anzeigemittel (11), die von den Datenverarbeitungsmitteln (10) gesteuert werden, und
- Speichermittel (10.3) zum Speichern einer Druck-Volumen-Kurve der Compliance des Kreislaufs (C1), die die Compliance des Atemkreislaufs darstellt,
**dadurch gekennzeichnet, dass**:
- es ferner vom Benutzer betätigbare Auswahlmittel (11.2) umfasst, um eine Bestimmung des Atemwegsöffnungsdrucks (oder AOP-Drucks) zu starten,
- die Datenverarbeitungsmittel (10) so konfiguriert sind, dass sie eine Bestimmung des AOP-Drucks als Reaktion auf eine Betätigung der Auswahlmittel (11.2) durch den Benutzer durchführen:
a) indem sie die Gasversorgungsmittel (2) so steuern, dass das Atemgas mit einer sogenannten "niedrigen" Atemfrequenz zwischen 2 und 10 Atemzyklen pro Minute und mit einem sogenannten "langsamen" Einblasfluss zwischen 2 und 10 I/min zugeführt wird, bis ein Inspirationsvolumen zwischen 200 und 1500 ml oder ein maximaler Druck (Pmax) zwischen 20 und 60 cmH□O erreicht wird,
b) wobei auf den Anzeigemitteln (11) die gespeicherte Druck-Volumen-Kurve der Compliance des Kreislaufs (C1) und eine der Insufflation mit langsamem Fluss entsprechende Druck-Volumen-Kurve (C2) angezeigt werden, wobei die Druck-Volumen-Kurve (C2) der Insufflation mit langsamem Fluss überlagert zur gespeicherten Druck-Volumen-Kurve der Compliance (C1) des Kreislaufs angezeigt wird,
c) wobei auf der Druck-Volumen-Kurve (C2) der langsamen Insufflation einen Wendepunkt (PI) zu suchen und zu lokalisieren, der einer Änderung der Steigung der genannten Druck-Volumen-Kurve (C2) der langsamen Insufflation entspricht, wobei die Druck-Volumen-Kurve der langsamen Insufflation (C2) von der Druck-Volumen-Kurve der Compliance des Kreislaufs (C1) getrennt wird, und
d) durch Bestimmen des Druckwerts am auf der Kurve bei langsamem Durchfluss (C1) lokalisierten Wendepunkt (PI),
- und die Datenverarbeitungsmittel (10) ferner so konfiguriert sind, dass sie eine Anzeige des AOP-Druckwerts (20) auf den Anzeigemitteln (11) steuern, der dem auf der Kurve mit langsamem Durchfluss (C2) ermittelten Druckwert am Wendepunkt (PI) entspricht.

2. Beatmungsgerät nach Anspruch 1, **dadurch gekennzeichnet, dass** die Datenverarbeitungsmittel (10) ferner so konfiguriert sind, dass sie auf den Anzeigemitteln (11) die Anzeige einer Information über das Vorliegen eines Verschlusses der Atemwege des Patienten steuern.

3. Beatmungsgerät nach Anspruch 1, **dadurch gekennzeichnet, dass** die Anzeigemittel (11) einen Touchscreen (11.1) umfassen.

4. Beatmungsgerät nach Anspruch 1, **dadurch gekennzeichnet, dass** die Auswahlmittel (11.2) eine auf den Anzeigemitteln (11), insbesondere auf dem Touchscreen (11.1), angezeigte virtuelle Taste umfassen.

5. Beatmungsgerät nach Anspruch 1, **dadurch gekennzeichnet, dass** die Datenverarbeitungsmittel (10) ferner so konfiguriert sind, dass sie die Anzeige eines empfohlenen PEP-Drucks auf den Anzeigemitteln (11) steuern, wobei der empfohlene PEP-Druck aus dem ermittelten AOP-Druck (20) bestimmt wird.

6. Beatmungsgerät nach Anspruch 1, **dadurch gekennzeichnet, dass** der AOP-Druckwert (20) zwischen 1 und 30 cmH□O liegt.

7. Beatmungsgerät nach Anspruch 1, **dadurch gekennzeichnet, dass** die Gasversorgungsmittel (2) eine motorgetriebene Turbine (2.1) umfassen, die von den Datenverarbeitungsmitteln (10) gesteuert wird.

8. Beatmungsgerät nach Anspruch 1, **dadurch gekennzeichnet, dass** die Druckmessmittel (4) einen Drucksensor umfassen.

9. Beatmungsgerät nach Anspruch 1, **dadurch gekennzeichnet, dass** die Durchflussermittlungsmittel (5) einen Durchflusssensor umfassen.

10. Beatmungsgerät nach den Ansprüchen 1 und 5, **dadurch gekennzeichnet, dass** es ein PEP-Ventil (13) umfasst, das im Patientenkreislauf (3) angeordnet ist, und dass die Datenverarbeitungsmittel (10) so konfiguriert sind, dass sie den PEP-Druck des PEP-Ventils (13) auf den empfohlenen PEP-Druck einstellen.

11. Beatmungsgerät nach Anspruch 1, **dadurch gekennzeichnet, dass** die Datenverarbeitungsmittel (10) so konfiguriert sind, dass sie die Gasversorgungsmittel (2) so steuern, dass das Atemgas mit einer sogenannten "niedrigen" Atemfrequenz zwischen 3 und 7 Atemzyklen pro Minute und/oder mit einem sogenannten "langsamen" Flussrate zwischen 3 und 7 I/min zu liefern.

12. Beatmungsgerät nach Anspruch 5, **dadurch gekennzeichnet, dass** der empfohlene PEP-Druck höher ist als der ermittelte AOP-Druck (20).

13. Beatmungsgerät nach Anspruch 1, **dadurch gekennzeichnet, dass** die Gasversorgungsmittel (2) so konfiguriert sind, dass sie ein Atemgas liefern, das aus Luft, Sauerstoff und einem Luft-Sauerstoff-Gemisch ausgewählt wird.

14. Beatmungsgerät nach Anspruch 1, **dadurch gekennzeichnet, dass** die Gaszufuhrvorrichtungen das Atemgas mit der Einblasrate über einen Zeitraum zuführen, der ausreicht, um das gewünschte Inspirationsvolumen zwischen 200 und 1500 ml oder den gewünschten Maximaldruck (Pmax) zwischen 20 und 60 cmH□O zu erreichen.

15. Beatmungsgerät nach Anspruch 14, **dadurch gekennzeichnet, dass** die genannte ausreichende Dauer weniger als 60 Sekunden beträgt.

## Claims

1. A medical ventilator (1) comprising:
- gas supply means (2) for supplying a respiratory gas,
- a patient circuit (3) supplied with respiratory gas by the gas supply means (2),
- pressure measurement means (4) configured to determine the gas pressure in the patient circuit (3),
- flow determination means (5) configured to determine the flow rate of respiratory gas in the patient circuit (3),
- data processing means (10) comprising at least one processor (10.1) for processing the pressure and/or flow rate measurements taken by the pressure measurement means (4) and the flow rate determination means (5),
- display means (11) controlled by the data processing means (10), and
- storage means (10.3) for storing a circuit compliance pressure/volume curve (C1) representing the compliance of the respiratory circuit,
**characterized in that**:
- it further comprises selection means (11.2), operable by the user to initiate a determination of the airway opening pressure (or AOP pressure),
- the data processing means (10) are configured to perform a determination of the AOP pressure in response to user activation of the selection means (11.2):
a) by controlling the gas delivery means (2) to deliver respiratory gas at a so-called "low" respiratory rate of between 2 and 10 ventilatory cycles per minute, and at a so-called "slow" flow rate of between 2 and 10 L/min, until an inspiratory volume of between 200 and 1500 mL or a maximum pressure (Pmax) between 20 and 60 cmH□O,
b) by controlling a display on the display means (11) of the stored circuit compliance pressure-volume curve (C1) and a pressure-volume curve corresponding to the slow-flow insufflation (C2), the pressure-volume curve (C2) of the slow-flow insufflation being displayed superimposed on the stored pressure-volume compliance curve (C1) of the circuit,
c) by searching for and locating on the slow-flow insufflation pressure-volume curve (C2), an inflection point (PI) corresponding to a change in slope of said slow-flow inflation pressure-volume curve (C2) with separation of the slow-flow inflation pressure-volume curve (C2) from the circuit compliance pressure-volume curve (C1), and
d) by determining the pressure value at the inflection point (PI) located on the low-flow curve (C1),
- and the data processing means (10) are further configured to control a display on the display means (11) of the AOP pressure value (20) corresponding to the pressure value at the inflection point (PI) determined on the low-flow curve (C2).

2. Ventilator according to claim 1, **characterized in that** the data processing means (10) are further configured to control a display on the display means (11) of information relating to the existence of an airway closure in the patient.

3. Ventilator according to claim 1, **characterized in that** the display means (11) comprise a touch screen (11.1).

4. Ventilator according to claim 1, **characterized in that** the selection means (11.2) comprise a virtual button displayed on the display means (11), in particular on the touch screen (11.1).

5. Ventilator according to claim 1, **characterized in that** the data processing means (10) are further configured to control the display on the display means (11) of a recommended PEP pressure, said recommended PEP pressure being determined based on the AOP pressure (20) that has been determined.

6. Ventilator according to claim 1, **characterized in that** the AOP pressure value (20) is between 1 and 30 cmH□O.

7. Ventilator according to claim 1, **characterized in that** the gas supply means (2) comprise a motorized turbine (2.1) controlled by the data processing means (10).

8. Ventilator according to claim 1, **characterized in that** the pressure measurement means (4) comprise a pressure sensor.

9. Ventilator according to claim 1, **characterized in that** the flow determination means (5) comprise a flow sensor.

10. A ventilator according to claims 1 and 5, **characterized in that** it comprises a PEP valve (13) arranged on the patient circuit (3) and the data processing means (10) are configured to set the PEP pressure of said PEP valve (13) to the recommended PEP pressure.

11. Ventilator according to claim 1, **characterized in that** the data processing means (10) are configured to control the gas delivery means (2) to deliver respiratory gas at a so-called "low" respiratory rate of between 3 and 7 ventilatory cycles per minute and/or at a so-called "slow" flow rate of between 3 and 7 L/min.

12. Ventilator according to claim 5, **characterized in that** the recommended PEP pressure is greater than the determined AOP pressure (20).

13. Ventilator according to claim 1, **characterized in that** the gas supply means (2) are configured to supply a selected respiratory gas from among air, oxygen, and an air/oxygen mixture.

14. A ventilator according to claim 1, **characterized in that** the gas delivery means deliver the respiratory gas at the insufflation flow rate for a duration sufficient to achieve the desired inspiratory volume of between 200 and 1500 mL or the desired maximum pressure (Pmax) of between 20 and 60 cmH□O.

15. A ventilator according to claim 14, **characterized in that** said sufficient duration is less than 60 seconds.
